# EUROPEAN PATENT APPLICATION

(11) **EP 4 345 743 A1**
(43) Date of publication of application: **03.04.2024**
(21) Application number: 23197132.6
(22) Date of filing: 13.09.2023
(51) Int. Cl.: G06T 7/00, G06T 7/11

(54) **MEDICAL IMAGING DATA NORMALIZATION FOR ANIMAL STUDIES**

(30) Priority: 15.09.2022 US 202217932311
(71) Applicant: Siemens Healthineers AG, 91301 Forchheim (DE)
(72) Inventor: GEIGER, Bernhard, Cranbury, NJ, 08512 (US)
(74) Representative: Horn Kleimann Waitzhofer Schmid-Dreyer Patent- und Rechtsanwälte PartG mbB

(57) **Abstract**

Systems and methods that normalize medical imaging data (160) between different species and breeds of animals in order to allow for cross-species and crossbreed usage of machine trained models (195). Image data of a non-human subject is acquired, registered using a standardized model (190), and segmented using a machine trained model.

## Description

### FIELD

This disclosure relates to medical imaging processing.

### BACKGROUND

Advanced medical treatments that have previously only been available to humans have now been implemented for use in other animals such as pets (cats, dogs, birds, farm animals etc.). These treatments for non-human animals are similar in implementation to treatment for humans. In the example of radiation treatment, internal organs of the subject are identified, and a treatment plan is calculated to maximize radiation dose in the target and minimize it in the healthy organs. This planning, in particular the delineation of organs at risk, is typically provided by neural networks that are configured with machine learning and annotated data. The process is similar for non-human animals as it is in humans except that a different network must be used that is trained on data specific to the species or breed of animal.

In practice, a neural network is input a large number of training data for the species or breed of animal and learns how to recognize the structures that were annotated. However, the performance of the neural network depends on the amount and range of training data. Due to the difference in size and locations of organs and anatomy between different breeds and species, acquiring a sufficient amount of training data and properly training networks may be difficult and inefficient. An accurate and effective machine learned network would have to be trained with a large amount of annotated training data for each distinct breed and/or species, which is a considerable effort. In addition, size differences between animals that are the same breed may hamper the training process.

### SUMMARY

By way of introduction, the preferred embodiments described below include methods, systems, instructions, and computer readable media for medical imaging data normalization for animal studies.

In a first aspect, a system for medical imaging data normalization for animal studies is provided. The system includes a medical imaging device, a memory, and a processor. The medical imaging device is configured to acquire image data for a non-human subject. The memory is configured to store a standard model and a machine trained network for segmentation of image data. The processor is configured to register the image data to the standard model and segment the registered image data using the machine trained network, the processor further configured to warp the segmented registered image data to the image data and generate an output image for the non-human subject based on the warped segmented registered image data.

The medical imaging device comprises an MRI device, a CT device, a cone-beam CT, an X-ray device, or a PET device. In an embodiment, the non-human subject comprises a canine and the standard model comprises an average anatomical model for a plurality of different breeds of canines.

In an embodiment, the processor is configured to register the image data by deforming a scale and a location of the image data to match one or more landmarks shared by the image data and standard model.

In an embodiment, the processor is configured to segment the image data into one or more regions of interest, wherein each region of interest is registered to a respective standard model of a respective region.

In an embodiment, the output image of the machine trained network is used to adjust a dose of radiation for radiation treatment. The system may include a display configured to display the output image for the non-human subject.

In a second aspect, a computer implemented method is provided comprising: acquiring image data for a non-human subject; registering the image data to a standardized model; identifying one or more features in the registered image data using a machine learned model; and providing the one or more features to an operator.

The non-human subject may be a dog, wherein the standardized model is generated from a plurality of breeds of dogs including multiple size variations.

The image data is acquired by an MRI device, a CT device, a cone-beam CT, an X-ray device, or a PET device.

In an embodiment, registering the image data comprises deformable registration.

In an embodiment, the one or more features comprise a location and classification of an organ of the non-human subject. The one or more features may be used to generate a plan for radiation treatment. In an embodiment, the method further includes implementing the plan.

In a third aspect, a method is provided for generating training data, the method including: acquiring image data for a first non-human subject; deforming the image data to fit a model of a second non-human subject; training a network to generate one or more predictions when input new image data, wherein the training uses the image data, the deformed image data, and respective annotations as training data; and storing the network for use in feature detection for a medical imaging procedure of a non-human subject.

The first non-human subject and the second non-human subject may be different breeds of a same species.

In an embodiment, deforming the image data comprises scaling or warping the image data so that one or more shared landmarks in the image data and model are aligned.

In an embodiment, the image data is acquired using an MRI system and the medical imaging procedure is for radiation treatment of the non-human subject. In embodiments, the method according to the second and/or third aspect is carried out by a system according to the first aspect.

The present invention is defined by the following claims, and nothing in this section should be taken as a limitation on those claims. Further aspects and advantages of the invention are discussed below in conjunction with the preferred embodiments and may be later claimed independently or in combination.

It is understood that, in embodiments, the system of the first aspect is implemented to carry out a method according to the second and/or third aspect.

### BRIEF DESCRIPTION OF THE DRAWINGS

Figure 1 depicts an embodiment of a system for medical imaging data normalization for animal studies.
Figure 2 depicts an example workflow for medical imaging data normalization for animal studies.
Figure 3 depicts an example workflow for medical imaging data normalization for animal studies.
Figure 4A depicts an example of an image of non-human anatomy.
Figure 4B depicts an example of an image of human anatomy.
Figure 5 depicts an example workflow for generating a network trained using deformed image data.

### DETAILED DESCRIPTION

Embodiments described herein provide systems and methods that normalize medical imaging data between different animals in order to reduce the required effort for annotation, and also allow for cross-species usage of machine learned models. Normalization of the medical imaging data may also be used to augment available training data for different species or breeds for which there is limited available data.

Anatomical structure detection is a fundamental task in medical image analysis that involves computing the location information of organs and/or landmarks in two-dimensional or three-dimensional image data. Localized anatomical information can guide more advanced analysis of specific body parts or pathologies from the images, for example organ segmentation, lesion detection, and radiotherapy planning. Detection of abnormalities (for example, including tumors and other suspicious growths) in medical images is a common but costly and time-consuming task. Given that the location is often not known beforehand, an operator may search across the two-dimensional image or three-dimensional volume to find deviations compared to surrounding tissue and then to determine whether that deviation constitutes an abnormality that requires follow-up procedures or something that can be dismissed from further investigation. This is often a difficult task that can lead to errors in many situations either due to the vast amount of data that needs to be searched to find the abnormality (e.g., in the case of volumetric data or whole-slide images) or because of the visual similarity of the abnormal tissue with normal tissue (e.g., in the case of low-contrast lesions in mammography). This task is further complicated by the different anatomies for different species and breeds. Automated detection systems have therefore been of great interest in the research community for many years due to their potential for reducing reading costs, shortening reading times and thereby streamlining the clinical workflow.

Machine learned models are used in feature detection and/or segmentation in many imaging implementations. For example, machine learned models may be used in radiation oncology to assist in treatment planning, assess response to therapy, and provide automated adaptation in treatments over time. Radiation therapy, in particular, is one treatment that makes use of machine learned models to improve the process and the outcome. Radiation therapy is the use of high-energy radiation to damage cancer cells' DNA and destroy their ability to divide and grow. The high-energy radiation may be delivered using machines called accelerators or via radioactive sources placed inside the patient on a temporary or permanent basis. Because radiation therapy can damage normal cells, medical imaging-such as x-ray, computed tomography (CT) or magnetic resonance imaging (MRI)-may be used to help precisely target the radiation dose to the cancer. In an example, MRI-guided radiation therapy uses magnetic resonance imaging (MRI) together with radiotherapy to treat tumors and cancers throughout the body. The use of MRI during radiotherapy enables the most precise targeting of radiation by accurately identifying the position and shape of a patient's tumor and other structures / organs / features of interest.

However, machine learned models are only as good as the data on which the models / networks are trained. In an example operation a network inputs training data and outputs a prediction. The prediction is compared to the annotated training data. A loss function may be used to identify the errors from the comparison. The loss function serves as a measurement of how far the current set of predictions are from the corresponding true values. During training and over repeated iterations, the network attempts to minimize the loss function as the result of a lower error between the actual and the predicted values means the network has done a good job in learning. The process of inputting, outputting, comparing, and adjusting is repeated for a predetermined number of iterations with the goal of minimizing the loss function. This process is driven by the training data and more specifically a relationship between the contents of the training data and the provided annotations. As an example, a machine learned model may learn to identify a passenger vehicle given images of a passenger vehicle and accurate annotations. The machine learned model, however, would be unable to identify a bicycle, bus, truck, etc. if that data was not included in the training dataset. Similarly, in the medical imaging field, the training data must be related to and sufficient enough for a machine learned model to learn its appointed task. For humans, there is generally a sufficient amount of training data for certain medical tasks. For example, while there may be some variation between patients, a human liver of any patient is similar enough to other human livers so that a machine learned model trained using images of human livers can segment and identify a human liver in an unannotated image. A large number of such imaging procedures are performed each year and a sufficient number of training data samples may be generated or collected. This, however, is not the case for other species. Medical imaging studies are not as common for non-human animals such as pets (dogs, cats, birds, reptiles, etc.) and as such, there may not be a sufficient amount of training data to train a model. In addition, variation between species and between interspecies breeds may further limit the training data that is required for each model. Further, as a different model may be required for each species or breed, the number of total models may be unwieldy.

Embodiments provide normalization and registration so that fewer models / training data may be used for medical imaging analysis for different species / breeds. In an embodiment, an intermediate model is generated that approximates an average sized animal. Image data is registered to match the intermediate model. Such a registration may use deformable registration, landmark based registration, or affine registration among other registration methods. In another embodiment, the body of the subject is separated into regions of interest for each organ. While it may be difficult to deform the complete body to match a model, it may be more successfully done for each organ + typical surrounding. For example, a canine kidney might sit at a different position in a particular animal, but once it is isolated with some amount of surrounding tissue and, for example scaled, the canine kidney may be matched to a different canine or even a human kidney. In this way, extensive work for human organ recognition may also be transferred for use in non-human animals. Extractions of organs + surroundings may also be based on landmark models that are much easier to annotate and train. In another embodiment, for different deformations (landmark based or based on a simple template of different breeds), annotations may be augmented so that breeds that were not in the original training sets may be recognized. Averaging between models can be used to train potential crossbreeds. The augmented data provides synthetic training data that can be used to provide better trained models or networks.

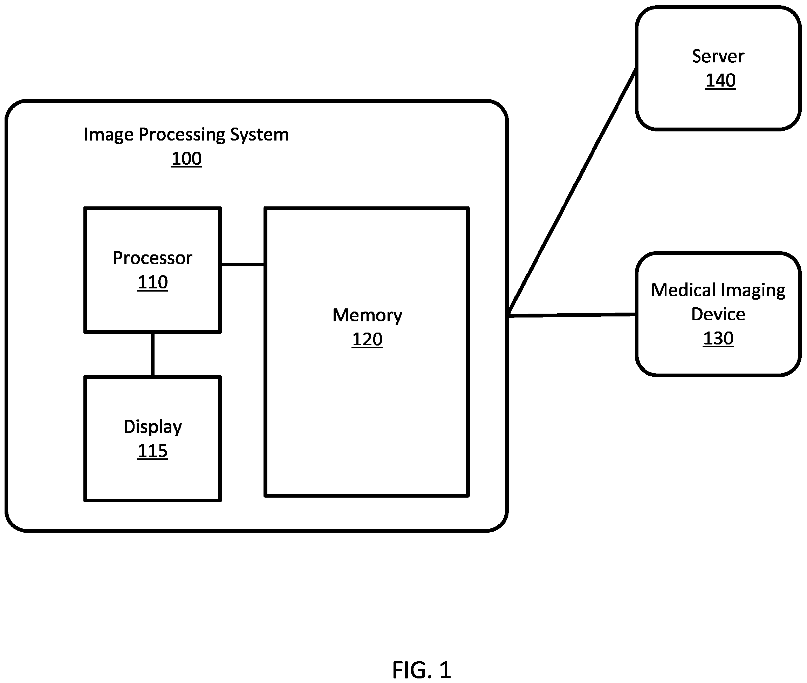

Figure 1 depicts a system for medical imaging data normalization for animal studies. The system 100 includes an imaging processing system 100, a medical imaging device 130, and optionally a server 140. The imaging processing system 100 includes a processor 110, a memory 120, and a display 115. The imaging processing system 100 may be includes with or coupled to the medical imaging device 130. Certain imaging functions may be performed by any of the imaging processing system 100, the medical imaging device 130, or the server 140. The imaging processing system 100 may be configured to train or store a machine learned model / network that is used for extracting or detecting features from non-human imaging procedures performed using the medical imaging device 130. Image data may be acquired from the medical imaging device 130 in real time or may be generated, stored, and processed at a later time.

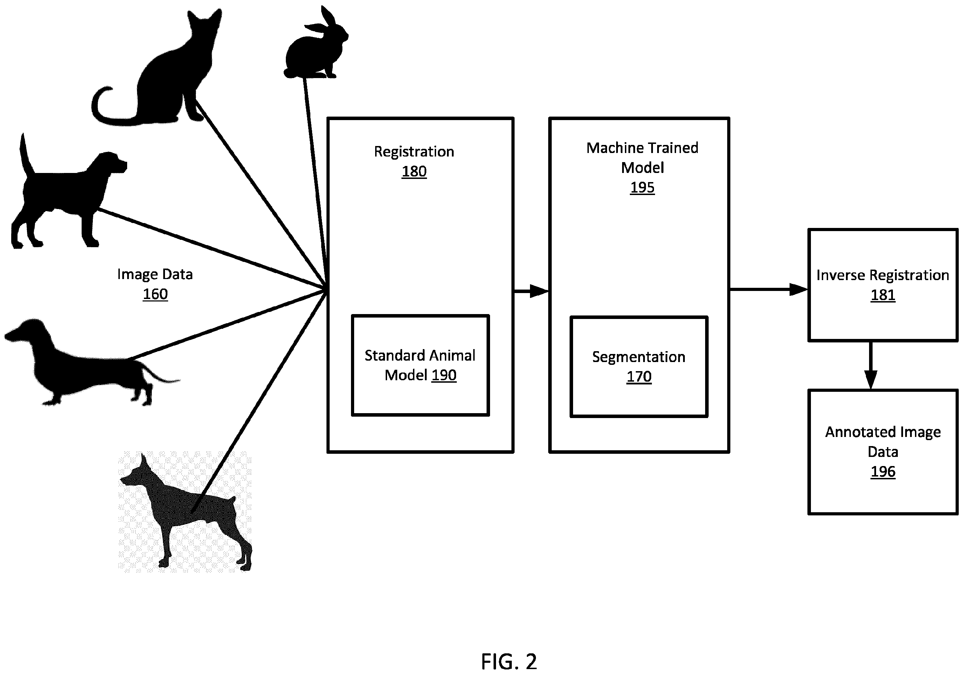

Figure 2 depicts an example of the workflow for medical imaging data normalization. Image data 160 for multiple species of animals / breeds are acquired then registered 180 using a standard animal model 190. The output may then be used for feature extraction / segmentation 170 / identification / analysis by a machine trained model 195 as referred to as a neural network 195 or just network 195. In an embodiment, the output is warped back (inverse registration 181) to align with the original image data 160 to generate annotated image data 196. In an example for a dog, image data for the dog is first registered to a standard animal model (there are different registration techniques, some work without any segmentation, some require landmarks, both can be deformable or affine (not rigid)). Then a trained network 195 is used to segment the organs of the registered data. Then the organs are then warped back to match the input image data 160 for the dog so that, for example, a treatment plan for the dog may be generated or further processing may be performed. In the example of a treatment plan, the segmented image data (tissue / organ / etc.) may be used for location identification, volume measurements, change detection, quantification, etc. Further processing may be performed on the segmented image data.

For the medical imaging device 130, one example used herein is in a MR context (i.e., a MR scanner), but other types of scanner may be used (e.g., reconstruction for CT, PET, SPECT, or other medical imaging). The MR scanning device 130 is only exemplary, and a variety of MR scanning systems can be used to collect the MR data. The MR scanner 130 is configured to scan a non-human subject. The scan provides scan data in a scan domain. The medical imaging device 130 scans a patient to provide k-space measurements (measurements in the frequency domain). An image reconstruction process is used to generate an image from the k-space measurements.

In another example, the medical imaging device 130 is or includes a CT system. The CT system includes an x-ray source and opposing detector mounted in a gantry. The CT system is an x-ray scanner configured to obtain attenuation data (e.g., measures of tissue density in Hounsfield units) for a patient volume. The gantry moves the source and detector about the patient for scanning. The processor 110 or a different processor computes the attenuation of the x-rays at different voxels within the scan volume. Any now known or later developed CT system may be used. Other x-ray scanners, such as a CT-like C-arm scanner, cone-beam CT scanner, or x-ray device may be used.

In another example, the medical imaging device 130 is a PET system. The PET system is a nuclear imaging system. The detectors detect gamma rays emitted indirectly by a positron-emitting tracer. Pairs of gamma rays generated by a same positron annihilation event may be detected using the ring of the detectors. The pairs of gamma rays travel about 180 degrees apart. If the direction of travel intersects the arrangement of detectors 15 at two locations, a coincident pair may be detected. To distinguish specific pairs, the coincidence of detected gamma rays is determined. The timing of receipt is used to pair the detected gamma rays. The timing, as prompt data, may also indicate the time of flight (TOF), providing information about where along a line of response the emission occurred. Each individual detection output from the detectors includes energy, position, and timing information. Alternatively, the detectors output energy information and a receiving coincidence processor determines the timing and position (e.g., based on port assignment or connections). The timing information is used to determine coincidence of detection by different detectors by the coincidence processors with or without also determining a general position along the LOR of the emission based on TOF. Pairs of gamma rays associated with a same positron emission are determined. Based on the detected event, a LOR is determined from the detectors involved in the detection of that event. The detected events are passed to the memory 120 and/or processor 110. The processor 110 connects with the detectors, such as through the coincidence processors.

The medical imaging device 130 is configured to generate imaging data or medical images of a non-human subject. The term non-human as used herein describes an animal such as a dog, cat, rabbit, bird, reptile, etc. For a procedure, the non-human animal is typically sedated and laid on a bed. The bed slides or moves to a chamber which performs the medical imaging procedure. The bed is a gurney, table, or other support to hold an examination subject, such as a patient. A robot, gears, cable, track, and/or other device may move the bed between a position for scanning.

The imaging data or the medical image is data representing a two-dimensional slice or a three-dimensional volume of the subject. The data may be in any format. While the terms image and imaging are used, the image or imaging data may be in a format prior to actual display of the image. For example, the medical imaging data may be a plurality of scalar values representing different locations in a Cartesian or polar coordinate format different than a display format. As another example, the medical image may be a plurality red, green, blue (e.g., RGB) values output to a display for generating the image in the display format. The medical image may be currently or previously displayed image in the display or another format. The imaging data is a dataset that may be used for imaging, such as scan data or a generated image representing the patient.

The medical imaging data represents a two or three-dimensional region of the patient. For example, the medical imaging data represents an area or slice of the patient as pixel values. As another example, the medical imaging data represents a volume or three-dimensional distribution of voxels. The three-dimensional representation may be formatted as a stack or plurality of two-dimensional planes or slices. Values are provided for each of multiple locations distributed in two or three dimensions. The medical imaging data is acquired as one or more frames of data. The frame of data represents the scan region at a given time or period. The dataset may represent the area or volume over time, such as providing a 4D representation of the patient.

The medical imaging data or medical image is processed by the image processing system 100. The image processing system 100 includes a processor 110, display 115, and memory 120. The image processing system 100 may receive or transmit data to and from the server 140 that may also be configured to process the image or store data for future image processing or training / storage of machine trained models.

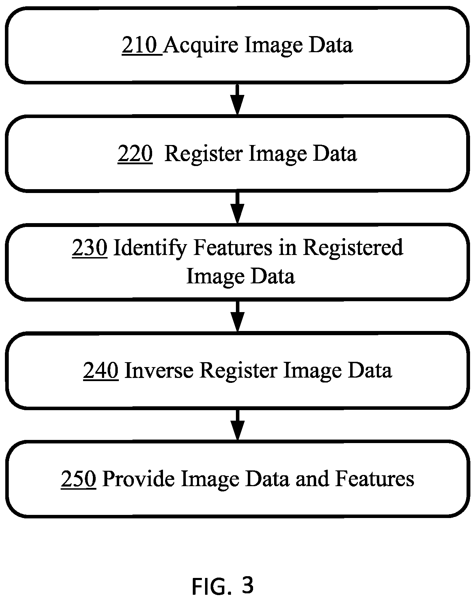

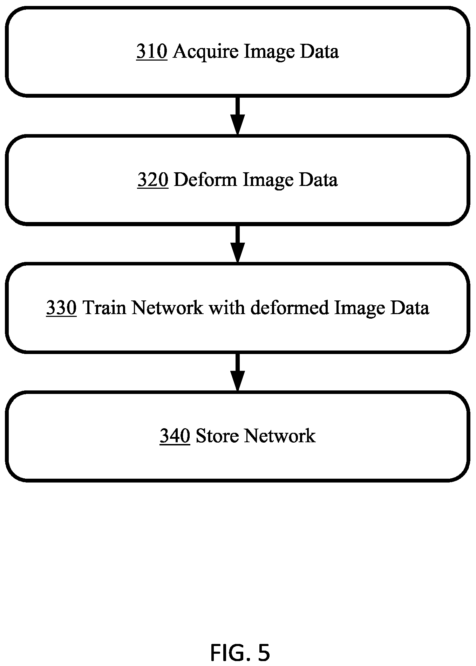

The processor 110 is a general processor, digital signal processor, graphics processing unit, application specific integrated circuit, field programmable gate array, artificial intelligence processor, digital circuit, analog circuit, combinations thereof, or other now known or later developed device for processing images, normalizing image data, registering image data, augmenting image data, among other steps described below. The processor is a single device, a plurality of devices, or a network. For more than one device, parallel or sequential division of processing may be used. Different devices making up the processor 110 may perform different functions, such as one processor for segmenting the image and one processor for implementing the machine learned model 195. In one embodiment, the processor 110 is a control processor or other processor of the medical imaging device 130. In other embodiments, the processor 110 is part of a separate workstation or computer. The processor 110 operates pursuant to stored instructions to perform various acts described herein. The processor 110 is configured by software, design, firmware, and/or hardware to perform any or all of the acts of Figures 3 and 5.

The processor 110 is configured for feature segmentation and feature extraction. The processor 110 is configured to register the image data to a standardized model 190. Any method for registration may be used. For example, deformable registration, landmark based registration, or affine registration may be used. Deformable image registration (DIR) involves estimating the geometric transformation between two images to map them onto a common coordinate system (CCS). The process is deformable, or nonlinear, because the estimated transformation does not include only rigid transformations (i.e., translation and/or rotation) but also deformations (e.g., shrinking or stretching). Landmark registration involves where images are spatially aligned by selecting control points from both the images and applying geometric transformation that helps in translation, rotation, scaling and shearing. Affine registration is any transformation that preserves collinearity (i.e., all points lying on a line initially still lie on a line after transformation) and ratios of distances (e.g., the midpoint of a line segment remains the midpoint after transformation).

The standard model 190 may be generated or provided by the image processing system 100. In an embodiment, the standard model 190 is generated by identifying an average sized or scaled anatomy of a non-human animal. For example, the standard model 190 may be based on an average sized dog (across multiple breeds). In another example, the standard model 190 may be generated from a human organ thus allowing the use of additional training data from humans to be used in configuring a machine learned model 195 for feature extraction and analysis. In another example, the standard model 190 may be selected as a model for which the deviation of organ location and scaling is minimized. The standard model 190 may be a model for multiple different species. The anatomy of certain species may be similar enough so that a single model may be used. Alternatively, multiple standard models 190 may be provided for disparate species such as between dogs and birds. The standard model 190 may be stored in the memory 120.

In an embodiment, shape model-based methods make use of the standard model 190 and attempt to find the best match of the standard model 190 to the data in a new image data using deformation or other registration techniques. In an example, landmark points, such as surface mesh indices or labels, are identified. The landmark points are points that may be easy to identify and mark in each image. The standard model 190 uses the landmark points to generate one or more expected images shapes for the model. For example, machine learning is used to generate the shape model.

The standard model 190 may be provided of the entire body or, for example, an organ or an object in the image data. The standard model 190 may be derived or trained from previous scans. An object is described by points, referred to as landmark points. The landmark points may be determined in a set of training images. From the landmark points, a point distribution model is constructed. Principal component analysis (PCA) may be applied to the point in the point distribution model to fit the model. A standard model 190 for an organ, e.g., an average / standard shape of the organ, may be stored in a memory 120 or acquired from other sources such as medical record database or PACS.

The standard model 190 may include labels for landmark or key points. A landmark may be, for example, an anatomical landmark. Anatomical landmarks may include points on or in an anatomical structure, for example, an organ, that may be present in similar organs from different patients. For example, general anatomy divides a liver into two portions, right and left lobes. Anatomical landmarks or regions for a liver include the division between the lobes, e.g., the falciform ligament. Further, a line may be drawn running from the left of the vena cava and all the way forward to divide the liver and gallbladder into two halves. Other anatomical landmarks exist, such as the ligamentum venosum and the round ligament of the liver (ligamentum teres), that further divide the left side of the liver in two sections. The porta hepatis, also known as the transverse fissure of the liver, divides this left portion into four segments. Each of these anatomical landmarks exist in livers from different subjects and as such may be used to define an average shape model for a specific type of organ. The average standard model 190 for an organ may include a set of landmark points. Connecting the points may provide a wire frame model of the organ.

The standard model 190 may be used to identify the organ in the images by using one or more fitting functions. For image data, specific landmarks may be selected automatically or by a user. Image data may not contain each of the landmarks for an organ. For example, different views of an organ may block out landmarks on the opposite site of the organ. As such, only the landmarks that are visible in the image data may be used.

The output of the registration is a transformation of the input image data from the medical imaging device 130 to a new dataset that corresponds to a coordinate system from the standard model 190, for example where landmarks, features, or other points are now matched from the image data to the standard model 190.

The processor 110 is configured to segment the registered image data using a machine learned model 195. The processor 110 may also be configured to identify one or more features in the registered image data using the machine learned model 195. The one or more features may include a class or type of tissue or organ, a lesion, an artifact, an abnormality, or other feature that is useful or relevant for treatment or a diagnosis. The machine learned model 195 may be any type of model.

Image segmentation extracts or identifies regions of interest (ROI) through a semiautomatic or automatic process. Segmentation divides an image into areas based on a specified description, such as segmenting body organs/tissues in the medical applications for border detection, tumor detection/segmentation, and mass detection. Segmentation may also be used for other tasks such as detecting pathologies, not just healthy organs. For example, a cancer site, or a cyst. Other uses may include segmentation for quantitative measurements like size, change, or local texture / density distributions. Any method for segmentation may be used. For example, segmentation may be thresholding-based, region-based, shape-based, model based, neighboring based, and/or machine learning-based among other segmentation techniques. Thresholding-based methods segment the image data by creating binary partitions based on image attenuation values, as determined by the relative attenuation of structures on the images. Region-based segmentation compares one pixel in an image to neighboring pixels, and if a predefined region criterion (e.g., homogeneity) is met, then the pixel is assigned to the same class as one or more of its neighbors. Shape-based techniques use either an atlas-based approach or a model-based approach to find a lung boundary. Model-based methods use prior shape information, similar to atlas-based approaches; however, to better accommodate the shape variabilities, the model-based approaches fit either statistical shape or appearance models of the lungs to the image by using an optimization procedure. Neighboring anatomy-guided methods use the spatial context of neighboring anatomic objects of the lung (e.g., rib cage, heart, spine) for delineating lung regions. In machine learning-based methods, the lung abnormalities and boundaries are predicted on the basis of the features extracted from the image data.

In an embodiment, the image data is segmented and separated into different organs / regions of interest. While it may be difficult to deform the complete body to match a full body model, it may be easier and more efficient to deform or fit individual organs or specific regions. For example, a canine kidney might sit at a different position in a particular animal, but once it is isolated with some amount of surrounding tissue and possibly scaled, it may be matched to a different canine or even a human kidney. In this way, extensive work for human organ recognition may be transferred for use in animals. Extractions of organs + surroundings could be based on artificial intelligence landmark models that are much easier to annotate and train.

Any of various machine-learned models may be used, such as a neural network 195 or support vector machine. In an embodiment, the machine learned model 195 may be configured as a classifier that is trained to classify pixels, voxels, or regions as a type(s) of tissue, part of an organ, etc. The machine learned model 195 may be trained using supervised or unsupervised learning. The machine learned model 195 may include a neural network 195 that is defined as a plurality of sequential feature units or layers. Sequential is used to indicate the general flow of output feature values from one layer to input to a next layer. Sequential is used to indicate the general flow of output feature values from one layer to input to a next layer. The information from the next layer is fed to a next layer, and so on until the final output. The layers may only feed forward or may be bi-directional, including some feedback to a previous layer. The nodes of each layer or unit may connect with all or only a sub-set of nodes of a previous and/or subsequent layer or unit. Skip connections may be used, such as a layer outputting to the sequentially next layer as well as other layers. Rather than pre-programming the features and trying to relate the features to attributes, the deep architecture is defined to learn the features at different levels of abstraction based on the input data. The features are learned to reconstruct lower-level features (i.e., features at a more abstract or compressed level). Each node of the unit represents a feature. Different units are provided for learning different features. Various units or layers may be used, such as convolutional, pooling (e.g., max pooling), deconvolutional, fully connected, or other types of layers. Within a unit or layer, any number of nodes is provided. For example, 100 nodes are provided. Later or subsequent units may have more, fewer, or the same number of nodes. Unsupervised learning may also be used based on the distribution of the samples, using methods such as k-nearest neighbor.

Different neural network configurations and workflows may be used for or in the machine learned classification model such as a convolution neural network (CNN), deep belief nets (DBN), or other deep networks. CNN learns feed-forward mapping functions while DBN learns a generative model of data. In addition, CNN uses shared weights for all local regions while DBN is a fully connected network (e.g., including different weights for all regions of a feature map. The training of CNN is entirely discriminative through backpropagation. DBN, on the other hand, employs the layer-wise unsupervised training (e.g., pre-training) followed by the discriminative refinement with backpropagation if necessary. In an embodiment, the arrangement of the trained network 195 is a fully convolutional network (FCN). Other network arrangements may be used, for example, a 3D Very Deep Convolutional Networks (3D-VGGNet). VGGNet stacks many layer blocks containing narrow convolutional layers followed by max pooling layers. A 3D Deep Residual Networks (3D-ResNet) architecture may be used. A Resnet uses residual blocks and skip connections to learn residual mapping.

The training data used for configured or training the machine learned model 195 includes many instances of medical imaging data / medical images and the corresponding ground truth / annotations. Tens, hundreds, or thousands of samples are acquired, such as from scans of different species / breeds and/or by image processing to create further samples. The training data is normalized as described above using a standard model 190 of the subject or a standard model 190 of one or more regions or organs. In an embodiment, the training data is acquired from a standard sized or apportioned subject. In an example, the training data may be human image data. In this example, there is a plethora of training data due to the abundance of imaging procedures performed on a human. The standard model may be similar to a human model or be a human model. In another example, the training data may be image data from a common breed or species for which there is sufficient training data to sufficiently train the network 195. A computer (e.g., processor 110) machine trains the network 195. For example, the network 195 is machine trained using training data. In one embodiment, deep learning is used. The training learns both the features of the input data and the conversion of those features to the desired output. Backpropagation, RMSprop, ADAM, or another optimization is used in learning the values of the learnable parameters of the network 195 (e.g., the convolutional neural network (CNN) or fully connection network (FCN)). Where the training is supervised, the differences (e.g., L1, L2, mean square error, or other loss) between the estimated output and the ground truth output are minimized.

Machine learning is an offline training phase where the goal is to identify an optimal set of values of learnable parameters of the model that can be applied to many different inputs. These machine-learned parameters can subsequently be used during clinical operation.

The processor 110 is configured to warp or transfer the information from the segmentation / classifier back to the original image data. This reverse registration / inverse registration provides an output that is similar to the original image data but with additional information, for example, information about which tissues / organs that the pixels / voxels correspond to. In a simple example, if a landmark is originally registered to the standard model by moving up 3 spaces, then the reverse registration would move the landmark back down 3 spaces. This step, in other words, may reverse the deformation that is used during registration so that the output is similar if not exactly the same as the original image but, with, for example annotations indicating the tissue types, organ boundaries, lesions, etc. Any changes during the segmentation process to fix artifacts or other issues may be carried over during the inverse registration process.

The processor 110 is configured to output the features and / or annotated image data. The output may be stored for later use or used in real time. An operator may use the annotated image data to perform additional tasks such as further scans or to generate a treatment plan. In an example, the processor 110 may use identified organs or tissues to calculate the radiation dose delivered to the particular organs and ensure that it falls within safe limits. This step of the radiation process may involve the radiation oncologist, the physicist, and dosimetrist. Using the scan images the system 100 and or medical professionals work together to design the field of radiation therapy treatment. The focus of treatment planning is to deliver a high dose of radiation to the tumor while limiting the dose received by surrounding tissue that is normal. This helps preserve normal tissue and reduces side effects of treatment. Treatment plans are customized for each subject.

The image data, standard model 190, landmarks, segmented data, registered data, training data, machine-learned model, analysis, treatment plan, and/or other data may be stored in the memory 120. The data is stored in any format. The memory 120 is a buffer, cache, RAM, removable media, hard drive, magnetic, optical, database, or other now known or later developed memory. The memory 120 is a single device or group of two or more devices. The memory 120 is part of the image processing system 100 or a remote workstation or database, such as a PACS memory.

The memory 120 is additionally or alternatively a non-transitory computer readable storage medium with processing instructions. The memory 120 stores data representing instructions executable by the processor 110 for image normalization or by a processor 110 for machine training. The instructions for implementing the processes, methods and/or techniques discussed herein are provided on non-transitory computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The image processing system 100 may include a display. For example, the processor 110 displays the resulting image data, annotations, or analysis of the subject or object being scanned. The images are displayed on the display. The output of the machine trained model, for example annotations, classifications, or other data, may also be displayed. The display is a CRT, LCD, plasma, projector, printer, or other display device. The display is configured by loading an image to a display plane or buffer. The display is configured to display the reconstructed MR image of the region of the patient. The display may provide a graphical user interface (GUI) enabling user interaction with the image processing system 100. The system 100 may include an operator interface, formed by an input and an output. The input may be an interface, such as interfacing with a computer network, memory 120, database, medical image storage, or other source of input data. The input may be a user input device, such as a mouse, trackpad, keyboard, roller ball, touch pad, touch screen, or another apparatus for receiving user input. The input may receive a scan protocol, imaging protocol, or scan parameters. An individual may select the input, such as manually or physically entering a value.

Figure 3 depict an example workflow for a method for normalization of animal study image data. The system 100 generates an intermediate model for an average sized animal. Image data is normalized by registering the image data to the intermediate model. The normalized image data may be used to train a machine learned model 195 for feature extraction and analysis. The following acts may be performed by the imaging processing system 100, processor 110, server, medical imaging device 130 or a combination thereof. Additional, different, or fewer acts may be provided. The acts are performed in the order shown or other orders. The acts may also be repeated. Certain acts may be skipped or adjusted.

At act 210, the system 100 acquires image data of a non-human animal. A non-human animal may be any type of animal that is not a human, for example, a cat, dog, bird, reptile, horse, cow, etc. The image data may be acquired using any type of medical imaging device 130 such as an MRI system or CT system. The image data may be acquired in real time or may be acquired at any point prior to the steps describe herein. In an example, the image data is acquired for a medical imaging procedure by placing the non-human animal on a bed and performing a scan. The results are then processed to determine a treatment plan as described below. In another example, the image data is acquired and annotated for use as training data in training a machine learned model 195 for feature identification and analysis.

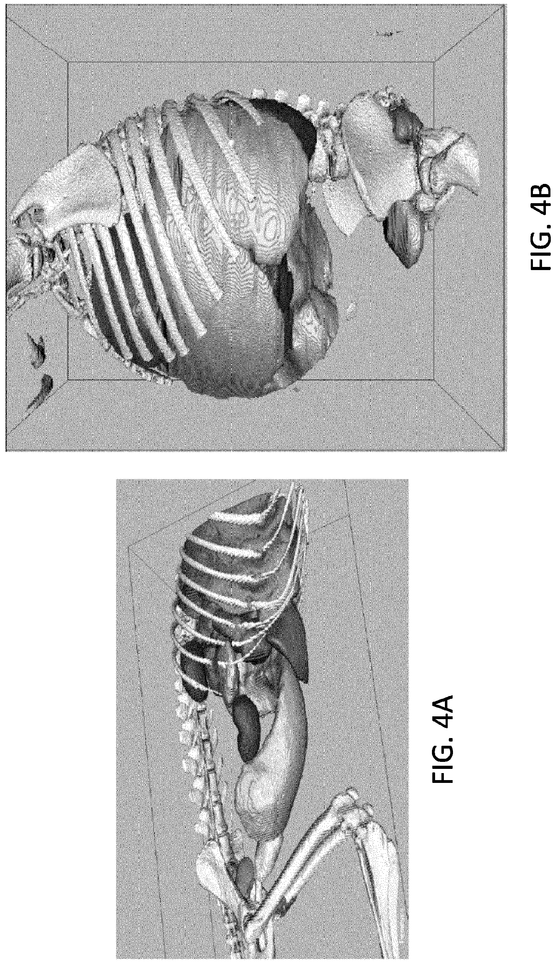

Figure 4A depicts an example of image data of a non-human animal. The image data may be compared to that of Figure 4B that depicts an example of a human anatomy. Different organs are shown using different colors. Both include the same or similar organs, but the location, orientation, and scaling of the organs / tissue / anatomy is different. Inputting the non-human image data into a model trained using human data would not provide accurate analysis.

At act 220, the system 100 registers the image data to a standardized animal model. The standardized animal model may be generated using an average or standard sized anatomy. The standardized animal model may be based on a human model so that the resulting registered image data may be processed by a machine learned model 195 that was training using human data. In an embodiment, the image data is segmented and separated into different organs / regions of interest which are then registered to different standardized models for different organs / regions of interest. Alternatively, a first region may be warped, then a second region warped, then a third region warped, etc. and then combined.

Image registration may include finding the spatial correspondence between the image data and the standardized model 190. The registration process may include deformation of the image data to fit the standardized animal model. Image registration can be categorized into two groups: rigid and non-rigid. Non-rigid image registration is also known as deformable image registration (DIR). In rigid image registration (RIR), all pixels move and/or rotate uniformly so that every pixel-to-pixel relationship remains the same before and after transformation. RIR may not be that successful in that the scale or location of the anatomy of the animal may be different than that of the standardized model 190. In deformable image registration, however, the pixel-to-pixel relationships change.

At act 230, the system 100 identifies one or more features in the image data using a machine learned model 195. The one or more features may include classifications of the pixels / voxels in the image data that are provided by a classifier implemented by the machine learned model 195. Embodiments provide a deep learning approach for creating a network 195 that cannot be practically performed manually. The deep learning approach is different than the approach taken by a human, such as through the definition of the architecture used. The disclosed networks may be implemented to computationally facilitate processing of medical imaging data and consequently improving and optimizing medical diagnostics and treatment. The machine learned model 195 may be any type of model that is configured to input an image and output features, classifications, treatments, diagnosis, etc. The machine learned model 195 may be trained on normalized data from any type of animal including different species or breeds as long as the anatomy of the animal (or organ) is similar to a point. For example, dogs, cats, rabbits etc. may share similar full body anatomy while differing from certain reptiles and birds. Organ specific models, however, may be shared between additional species, including humans.

The machine learned model may be configured to segment the registered non-human image data. Segmentation is the process of dividing an input into different parts or sections, e.g., for medical imaging, delineating the boundaries, or contours, of various tissues or structures in the body. Segmentation of organs and their substructures may be used to calculate clinical parameters such as volume, as well as to define the search region for computer-aided detection tasks to improve their performance. Any method of segmentation may be used. In an embodiment, one or more regions or organs are individually segmented. For example, after or during registration, the liver of the subject may be extracted along with surrounding information. The liver of the subject may be registered to a standardized model of the liver and then further processed. While it may be difficult to deform the complete body to match a full body model, it may be easier and more efficient to deform or fit individual organs or specific regions. In this way, extensive work for human organ recognition may also be transferred for use in animals. Extractions of organs + surroundings may use landmark models that are also easier to annotate and train.

At act 240, the segmented registered image data is warped back to align with the original image data. The warping back may include a reverse / inverse deformation of the segmented registered image data so that the output is similar to the image data but with the annotations / classifications / feature data provided by the machine learned model. In an example, image data is warped to a standard model. The warped data is segmented using a machine learned model that is trained using data that is similar to the standard model. The segmented data output from the machine learned model is then warped back to match the input image data.

At act 250, the system 100 outputs the image data with the annotations / classifications / feature data provided by the machine learned model. The image data and the annotations / classifications / feature data may be further processed, used for performing additional procedures, or for generating a treatment plan or information useful or relevant in generating a treatment plan for the subject that was scanned in act 210. During radiation treatment planning, organs adjacent to the tumor may be identified in order to calculate the radiation dose delivered to those crucial organs and ensure that it falls within safe limits. The output of act 240 may be segmented image that provides delineations or annotations for the boundaries of organs. The image data with annotations may also be used to identify issues or provide a diagnosis.

One of the most important challenges when it comes to medical imaging data sets is to obtain data of a sufficiently large number of properly annotated cases. The bottleneck may be obtaining the images and also obtaining annotations and reference standards. The reference standard has to be of high quality, especially when used for training but also for performance evaluation. Obtaining high-quality image data, annotations, and reference standards are expensive and time-consuming.

In an embodiment, using different deformations (landmark based or based on a simple template of different breeds), annotations can be augmented so that breeds that were not in the original training set may be recognized. Averaging between models can be used to train potential crossbreeds.

Figure 5 depicts a method for generating training data for training a model for analysis of multiple different variations of animals. The following acts may be performed by the imaging processing system 100, processor 110, sever, medical imaging device 130 or a combination thereof. Additional, different, or fewer acts may be provided. The acts are performed in the order shown or other orders. The acts may also be repeated. Certain acts may be skipped or adjusted. The processes of acquiring image data, deforming, and training may be similar to the processes discussed above for Figure 1 and Figure 3. Additional steps may be performed, for example segmentation or identification of a standard model 190 or scale of a subject.

At act 310, the system 100 acquires image data for a first subject. The image data may be acquired using any type of imaging modality. The first subject is a non-human species. The first species may be a particular breed of animal.

At act 320, the system 100 deforms the image data to mimic a second subject. The second subject may be the same species as the first subject but a different breed. The second subject may differ in scale or shape from the first subject. The deformation of the image data may include scaling, stretching, rotating, shrinking, etc. of the image data to fit a model or scale of the second subject. In an example, the first subject may be a great dane. The second subject may be a poodle. The image data for the great dane may be deformed to mimic the scale of a standard poodle. In this way, the deformed image data may be used to train a network 195 to be able to identify features when given image data for a poodle.

At act 330, the system 100 trains a network 195 to generate one or more predictions when input image data of either the first species or the second species, wherein the training uses the image data, the deformed image data, and respective annotations as training data. In this way, the network 195 may be trained on data that approximates or mimics additional species or breeds that would otherwise not be in the training data set. Additional data may also be useful in that there may be limited training data available for non-human subjects. In an example, the system 100 may only have access to ten datasets to train the network 195. This may not be sufficient. The system 100 takes the ten datasets and deforms the datasets to generate one hundred or more datasets. This process can be used to augment imaging studies for animals by doing deformations that mimic different species in order to generate synthetic training data.

At act 340, the system 100 stores the network 195 for use in analyzing future imaging procedures. The network 195 may be used, for example, for planning radiation treatment for non-human animals. For radiation treatment, internal organs of the subject are identified, and a treatment plan is calculated to maximize radiation dose in the target and minimize it in the healthy organs. This planning, in particular the delineation of organs at risk, may be provided by the trained networks that are configured with machine learning at act 330.

Although the subject matter has been described in terms of exemplary embodiments, it is not limited thereto. Rather, the appended claims should be construed broadly, to include other variants and embodiments, which can be made by those skilled in the art. The disclosed methods can be computer-implemented methods.

## Claims

1. A system for medical imaging data normalization for animal studies, the system comprising:
a medical imaging device (130) configured to acquire image data for a non-human subject;
a memory (120) configured to store a standard model (190) and a machine trained network (195) for segmentation of image data; and
a processor (110) configured to register the image data to the standard model (190) and segment the registered image data using the machine trained network (195), the processor (110) further configured to warp the segmented registered image data to the image data and generate an output image for the non-human subject based on the warped segmented registered image data.

2. The system of claim 1, wherein the medical imaging device (130) comprises an MRI device, a CT device, a cone-beam CT, an X-ray device, or a PET device.

3. The system of claim 1 or 2, wherein the non-human subject comprises a canine.

4. The system of claim 3, wherein the standard model (190) comprises an average anatomical model for a plurality of different breeds of canines.

5. The system of any one of claims 1-4, wherein the processor (110) is configured to register the image data by deforming a scale and a location of the image data to match one or more landmarks shared by the image data and standard model (190); and/or the processor (110) is configured to segment the image data into one or more regions of interest, wherein each region of interest is registered to a respective standard model (190) of a respective region.

6. The system of any one of claims 1-5, wherein the output image of the machine trained network (195) is used to adjust a dose of radiation for radiation treatment.

7. The system of any one of claims 1-6, further comprising:
a display (115) configured to display the output image for the non-human subject.

8. A computer implemented method comprising:
acquiring (210) image data for a non-human subject;
registering (220) the image data to a standardized model (190);
identifying (230) one or more features in the registered image data using a machine learned model (195); and
providing (250) the one or more features to an operator.

9. The computer implemented method of claim 8, wherein the non-human subject is a dog.

10. The computer implemented method of claim 8 or 9, wherein the standardized model (190) is generated from a plurality of breeds of dogs including multiple size variations.

11. The computer implemented method of any one of claims 8 - 10, wherein the image data is acquired by an MRI device, a CT device, a cone-beam CT, an X-ray device, or a PET device.

12. The computer implemented method of any one of claims 8 - 11, wherein registering the image data comprises deformable registration.

13. The computer implemented method of any one of claims 8 - 12, wherein the one or more features comprise a location and classification of an organ of the non-human subject; and/or wherein the one or more features are used to generate a plan for radiation treatment.

14. The computer implemented method of claim 13, further comprising:
implementing the plan.

15. A method for generating training data, the method comprising:
acquiring (310) image data for a first non-human subject;
deforming (320) the image data to fit a model of a second non-human subject;
training (330) a network (195) to generate one or more predictions when input new image data, wherein the training uses the image data, the deformed image data, and respective annotations as training data; and
storing (340) the network (195) for use in feature detection for a medical imaging procedure of a non-human subject.

16. The method of claim 15, wherein the first non-human subject and the second non-human subject are different breeds of a same species.

17. The method of claim 15 or 16, wherein deforming (320) the image data comprises scaling or warping the image data so that one or more shared landmarks in the image data and model are aligned.

18. The method of any one of claims 15 - 17, wherein the image data is acquired using an MRI system and the medical imaging procedure is for radiation treatment of the non-human subject.
